# EUROPEAN PATENT APPLICATION

(11) **EP 1 617 224 A1**
(43) Date of publication of application: **18.01.2006**
(21) Application number: 05254476.4
(22) Date of filing: 18.07.2005
(51) Int. Cl.: G01N 33/68

(54) **De novo sequencing using tandem mass spectrometry**

(30) Priority: 16.07.2004 US 892870; 10.09.2004 US 938690
(71) Applicant: Agilent Technologies, Inc., Palo Alto, CA 94306 (US)
(72) Inventor: Joyce, Timothy H., Mountain View, CA 94040 (US); Boyce, Barry E., Wilmington, DE 19808 (US); Nicol, Gordon R., Middletown, DE 19709 (US); Liu, Hongbin, Wilmington, DE 19808 (US)
(74) Representative: Tollett, Ian

(57) **Abstract**

Multiple derivatization by chemical reactions of analytes for mass spectrometry enhances the use of MS techniques for analyzing protein samples, particularly when the sequence of a polypeptide is determined by tandem MS/MS. Accurate mass analysis techniques are described for use in sequencing polypeptides, together with the use of sequencing data in protein analysis. Lock mass ions have characteristic m/z ratios and are used in instrument calibration and mass analysis. An apparatus and method for calibrating a mass spectrometer (5) by internally introducing calibration lock mass ions at a post-source stage (41), (45) of the mass spectrometer is also provided. Lock mass ions mix with the derivatized polypeptide analyte ions prior to mass analysis.

## Description

The present invention relates to a method to perform a mass analysis of a polypeptide and to a method to determine a sequence of a polypeptide analyte.

Proteomics is the field of protein research that studies the large scale or global analysis of the protein complement of an organism (Aebersold and Mann, 2003, Nature 422:198). Proteomics is important in research, diagnostic, and clinical applications because information from various technical disciplines, including chemistry, genetics, cell imaging, and chip- or microarray-based protein or DNA analyses is related to cell function and physiology. In practice, proteomics requires detailed analyses of complex data for a large number of proteins in a short time period. Analysis of the mass of proteins and peptides is particularly useful in large scale proteomics analysis.

Mass spectrometry (MS) is a potentially valuable tool in proteomics because highly sensitive measurements of mass can identify some proteins by their amino acid sequence. (Aebersold and Goodlett, Chem. Rev. 101: 269-295, 2001; reviewed in Mann, et al., 2001, Ann. Rev. Biochemistry 70:437; Kinter and Sherman, Protein sequencing and Identification Using Tandem Mass Spectrometry, Wiley, NY, 2000). Because each amino acid or chain of amino acid residues can theoretically be detected by an accurate mass measurement a sufficiently accurate measurement identifies the individual amino acids. When the sample processing and MS techniques are highly accurate, the actual sequence of amino acids that form a polypeptide molecule can be identified. Further, if a highly accurate and reliable method detects a deviation from the known mass for an amino acid the deviation, can indicate that the amino acid has been modified. Detection of protein structure modifications is extremely important in proteomics research.

Mass spectrometry (MS) involves the analysis of ionized analytes in a gas phase using an ion source that ionizes the analyte, a mass analyzer that measures the mass-to-charge (M/Z) ratio of the ionized analytes, and a detector that registers the number of ions at each m/z value. The MS apparatus may also be coupled to separation apparatus to improve the ability to analyze complex mixtures. Further, MS instrument combinations can be made to enhance sensitivity and selectivity. In recent times, numerous improvements have been made in sample preparation and ionization techniques, which collectively pertain to the "ion source" region of the mass spectrometer. Atmospheric Pressure Ionization (API) techniques, such as Electrospray (ESI), Atmospheric Pressure Chemical Ionization (APCI), Atmospheric Pressure Photoionization (APPI) and Atmospheric Pressure Matrix Assisted Laser Desorption/Ionization (AP MALDI) are now commonly used to generate analyte ions from fluid samples. These techniques have improved the sensitivity of mass spectrometer systems by increasing the concentration of ionized analyte molecules that enter the mass spectrometer and reach the detectors downstream.

In electrospray sources, an analyte solution from a source apparatus, such as a liquid chromatography column, is ejected from a needle as a liquid stream. Instabilities in the liquid stream generated by nebulizing means such as a nebulizing gas, pneumatic assist and/or ultrasonic waves result in breakup of the stream into droplets, many of which bear electric charge as a result of the needle being at high potential with respect to surrounding conductors, or due to triboelectric effects. The charged droplets are desolvated by evaporation, freeing desolvated, ionized analyte molecules. The analyte ions are then directed into a mass spectrometer interface from which the constituent molecules are transported through one or more vacuum stages downstream to a mass analyzer. At the mass analyzer, the analyte ions are filtered and then detected.

Concurrent improvements in mass analysis techniques, such as Time-Of-Flight (TOF) and Magnetic Sector and Fourier Transform Ion Cyclotron Resonance (FTICR), have made mass assignment accuracies on the order of 1 to 10 ppm (parts per million or greater) feasible. However, this level of accuracy requires a level of instrument stability and repeatability that is not always attainable due to "drift" caused by fluctuations in ambient temperature, spectrometer chamber pressures, and applied voltages. To adjust to such drift, instruments are calibrated using masses that are known, using a process referred to as mass calibration. According to this technique, known compounds (herein referred to as "lock masses") having characteristic m/z ratios, are typically analyzed either in conjunction or sequentially with samples of unknown compounds ("analytes"). The resulting mass spectrum contains one or more internal calibration peaks corresponding to the m/z ratio of the lock masses that can then serve as a scale by which the masses of peaks corresponding to the unknown compounds can be measured. The use of the lock mass ions can be used for direct calibration of the instrument, for error detection in the measurement of certain analytes, for comparison to particular peaks or intensities in certain analytes, and for any qualitative or quantitative mass analysis or data processing step. Accordingly, when polypeptide species are being measure by mass analysis, the spectrum includes both peaks resulting from lock mass ions as well as peaks resulting from peptide fragments. The resulting spectra, therefore, may contain information that is useful to both calibrate an MS instrument, derive sequence information about the polypeptide analyte and to facilitate the mathematical analysis of the analyte, which may be accomplished either independently or together with calibration of the instrument.

In one conventional method of mass calibration, lock masses are mixed with the unknown sample in solution prior to ionization in the ion source. This conventional method suffers from contamination because the lock masses contaminate transfer lines and capillary tips, and suppress ionization efficiency of the sample compounds. At the high accuracy threshold required for distinguishing between large molecular-weight compounds such as polypeptides, slight instrument drift can produce erroneous results, requiring successive analyses at a high-throughput rate before large drift fluctuations materialize. At high-throughput rates, lock mass contamination becomes a more important issue because the residue of the lock mass left over from previous analysis runs may be difficult to eliminate before succeeding analysis runs take place.

External introduction of lock masses alleviates the effects of contamination. See U.S. Patent No. 6,207,954, EP No. 0 966 022. However, external introduction techniques, the analyte sample and the lock mass ions must be emitted from separate probes, reducing interaction between the lock mass and sample in solution and probe contamination, thereby requiring duplication of sample probes and injectors.

Given the inherent complexity of peptide fragmentation and the difficulties of MS spectral analysis, a combination of different methods for chemical derivatization of peptides has not been completely developed. For proteomics and analysis of complex mixtures of peptides, it is accepted that only very simple and extremely efficient chemical derivatization steps are compatible with proteomics. If any heterogeneity is introduced by the chemical reaction, the peptide samples become even more complex, thereby complicating the MS analysis and subsequent data processing. (Mann and Jensen, Nat. Biotech. 21:255-261, 2003). Therefore, although chemical derivatization is a known procedure for use in mass spectrometry, the use of multiple discrete derivatization techniques would be expected to introduce significant complexity and complication to a peptide mass analysis and the use *of de* novo sequencing for a complete determination of the linear amino acid sequence of a peptide is still difficult.

The present invention is a novel approach to chemical derivatization of polypeptides for analysis by mass spectrometry and the use of internally introduced lock mass ions prior to mass analysis. The invention includes both methods and compositions of matter and specifically encompasses multiple chemical derivatives, the use of multiple derivatives in concert with MS instrumentation introducing or creating lock mass molecules, improved data analysis techniques applied to derivatized polypeptide methods for determining the amino acid sequence of modified peptides, and methods and apparatus for the use of all of the above in mass analysis. In certain embodiments, the invention also enables new techniques for MS data analysis using spectral data, computer databases, and software and algorithms that use MS data to identify proteins, identify peptides or sequences of peptides, and that perform *de novo* sequencing of polypeptides using mass analysis of polypeptides and lock mass ions..

In some embodiments, the invention is comprised of at least two chemical reaction steps wherein each is a derivatization of a chemical group present in a polypeptide. This process may be referred to as multiple derivatization because at least two distinct labeling methods are performed. The chemical reaction steps performed in the laboratory can be performed in series or in parallel under the circumstances where the chemical reactions do not interfere either in modification of the peptide or in cross-reaction between reagents, in such a way that compromises the reaction or the derivatization of the analyte peptide. Derivatization is typically performed on a sample that has been or will be subjected to digestion to yield polypeptide fragments and typically has at least two chemical labeling steps: in a first step, polypeptides are derivatized following a digestion to establish a reactive terminus and to achieve a first derivative to assist in identification of individual residues. An example of a first derivatization step is a lysine derivatization such as the approach described by Peters, et al. (WO 03/056299).

In a second derivatization, polypeptides that have been derivatized by the first derivatization step, such as those derivatized at the lysines, including particularly the C-terminal lysines, are subjected to a second chemical derivatization that uniquely modifies a separate moiety from the first derivatization. An example of a second derivatization is the alkylation of carboxyl groups, for example a methylation of carboxyl groups of aspartic acid residues. The method of performing two derivatizations of peptide moieties is distinguished from the use of nuclear isotopes as mass tags or the use of two step chemical reactions that feature the use of protective groups that shield specific peptide moieties from a single chemical derivatization. The derivatization of lysine may occur following enzymatic digestion or chemical fragmentation of the polypeptide. This derivatization step may advantageously be performed before or after alkylation of carboxyl groups depending on the analyte or other experimental parameters.

In one embodiment, tryptic digestion of a polypeptide or protein sample is followed by a first derivatization that preferably labels a C-terminus residue of the tryptic fragment, typically the creation of an imidazole derivative of C-terminal lysines. The first or single derivatized polypeptide is reacted with a second derivatizing agent to yield an additional derivatization of polypeptide acidic residue side-chains at carboxyl groups.

Another application of the present invention is to identify variants or modifications of a protein or polypeptide analyte present in a sample. Many important physiological conditions are caused or accompanied by a modification of a protein or polypeptide that may be detected in a biological sample such as blood, urine, saliva, cerebrospinal, fluid, ascites, plasma, cell or tissue samples or extracts or other substance commonly used in analytical methods that contains a polypeptide derived from a patient. With these samples, an accurate experimental measurement of a protein or polypeptide analyte permits analysis and diagnosis based on a comparison of a measured mass spectral pattern of a polypeptide with a hypothetical or standard mass spectral pattern. The standard spectral pattern may represent either a normal analyte or an analyte that is known to represent a disease state or a known physiological condition, or a particular genotype of interest. In this embodiment, an experimentally derived sequence is compared to a standard or reference and the difference is correlated to a specific modification or alteration existing between the standard or reference and the patient analyte. The measured differential thereby identifies a mutation, polymorphism, splice rearrangement, deletion, substitution, or other post-translational modification such as phosphorylation, acetylation, oxidation, methlylation, gelation, glycosylation, etc.

In different embodiments, the source of lock mass ions may include different structures for intruding lock mass molecules and include photo-ionization, field desorptionionization, electron ionization and thermal ionization apparatus. Lock mass ions can be introduced internally into a tandem mass spectrometer wherein the tandem mass spectrometer typically has a first mass analyzer stage, a collision cell, and a second mass analyzer stage. The collision cell receives derivatized polypeptide analyte ions from the first mass analyzer and includes collision gas that fragments the derivatized polypeptide ions into derivatized daughter ions of smaller size. The first mass analyzer stage in the collision cell make the separate units are combined into a single apparatus. The ionization can occur prior to introducing the analyte and lock mass molecules into the ion optics or can occur within the ion optics. Accordingly, the lock mass ions can be ionized substantially in or near the downstream path of the derivatized polypeptide ions so that both derivatized polypeptide ions and lock mass ions travel along the same path and are subjected to mass analysis at or substantially at the same time.

A method for mass calibration of polypeptide analyte by tandem mass spectrometry includes using a collision cell and creating lock mass ions within the collision cell. In this embodiment, lock mass ions are introduced into the collision cell and are ionized within the collision cell. However, those of skill in the art will recognize that ionization can occur either within or outside the collision cell. As noted above, the lock mass ions can be created within the ion optics that transport polypeptide analyte daughter ions to a mass analyzer stage. In this embodiment, lock mass ions are either created by introduced lock mass ions into the ion optics or by ionizing lock mass molecules within the ion optics. Each method described herein includes the step of separately calibrating the mass analyzer using the detection of the lock mass ions together with mass analysis of a lock mass ions either alone or in combination with the polypeptide analyte.
A number of preferred embodiments of the present invention will now be described with reference to the drawings, in which:

Figures 1A and 1B are MS/MS spectra (MALD1/Q-TOF) of imidazole labeled peptide (SEQ ID NO: 1) GLQYLLEK that has been derivatized at the lysine residue and with methylation-of carboxylate groups. Peptide (SEQ ID NO: 1) GLQYLLEK was generated from tryptic digestion of beta crystallin (bovine eye lens).

Figures 2A and 2B are MS/MS spectra (MALDI/Q-TOF) of imidazole labeled peptide (SEQ ID NO: 2) CDENILWLDYK generated from tryptic digestion of pyruvate kinase (rabbit muscle).

Figures 3A and 3B are MS/MS spectra of imidazole labeled peptide (SEQ ID NO: 1) GLQYLLEK when both the carboxy-terminal lysine and the amino-terminal lysine were derivatized with imidazole generated from tryptic digestion of β-crystallin (bovine eye lens).

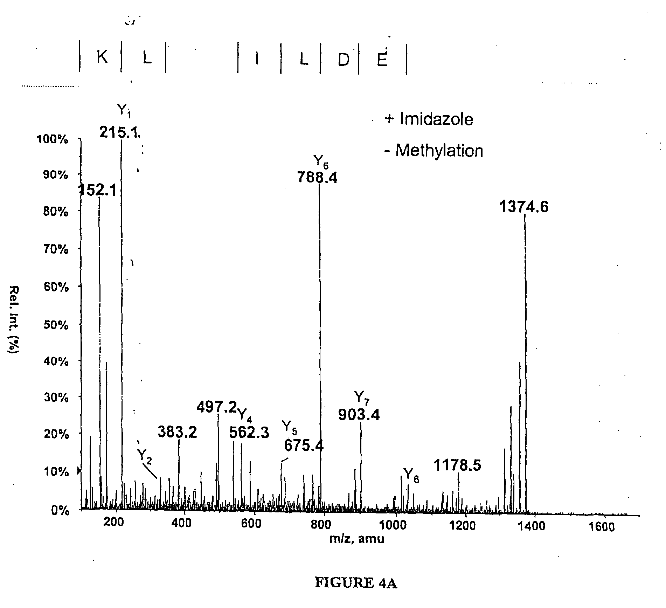

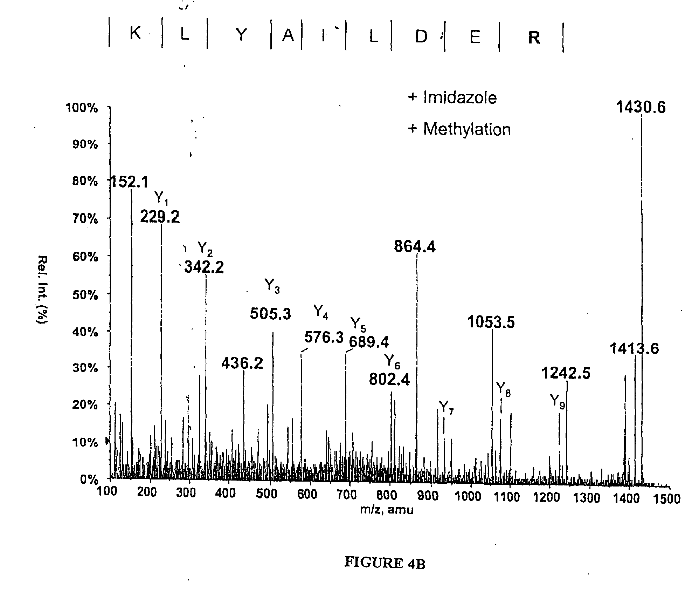

Figures 4A and 4B. Lys-C can be used to digest proteins to increase carboxy-terminal lysine occurrence, which could increase protein sequence coverage for identification. However, the resulting peptides after Lys-C digestion often have internal arginine, which make their MS/MS spectra difficult to interpret even after imidazole derivatization as shown in Figure 4A. The MS/MS spectrum of serially derivatized same peptide from cytochrome C (bovine heart) (SEQ ID No: 3) (Figure 4B) shows a long dominant y-ion series up to the internal arginine, permitting a read out of a long stretch of the peptide sequence.

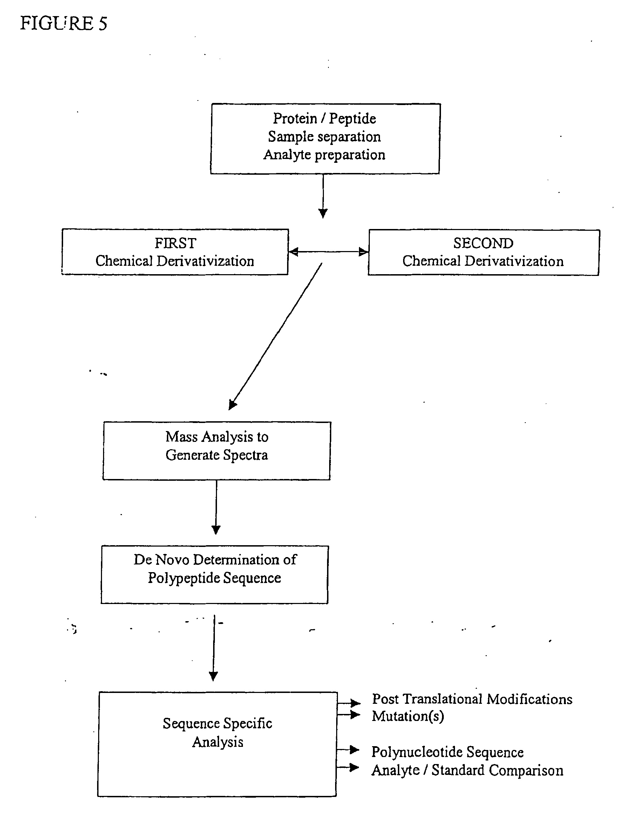

Figure 5 is an embodiment of the method of the invention including analysis of mass spectral data to perform a *de novo* peptide sequences analysis, to use sequence data in subsequent analysis and to perform any of a number of peptide analysis steps that require accurate sequence information.

FIG. 6 is a block diagram of a mass spectrometer system that incorporates an embodiment of the present invention.

FIG. 7 is a block diagram of the mass spectrometer system of FIG. 6 that incorporates an embodiment of the invention.

FIG. 8 illustrates an embodiment of the mass spectrometer system of FIG. 6 in which a concentric, coaxial radiation lamp is used as an ionization source.

FIG 9. illustrates an exemplary embodiment of a tandem mass spectrometer system (MS/MS) that incorporates the present invention.

FIG. 10 illustrates an embodiment of a tandem mass spectrometer system that incorporates the present invention.

Definitions:

As used herein, the terms "alkylating agent" refer to a compound capable of reacting with the carboxylate group of an amino acid to yield an alkyl group derivative as described herein.

The terms "mass analysis" refer to a process wherein the identification of an amino acid residue is determined by measurement of the mass to charge ratio (M/2).

"Polypeptide" refers to a polymer comprised of amino acid residues, related naturally occurring structural variants, and synthetic non-naturally occurring analogs thereof linked via peptide bonds, related naturally occurring structural variants, and synthetic non-naturally occurring analogs thereof. The term polypeptide also includes a plurality of amino acids existing as a cleavage, digestion, or fragmentation product of a larger polypeptide, wherein the cleavage, digestion, or fragmentation occurred by chemical, biochemical, ionization, mechanical or other reaction. The term "protein" typically refers to large polypeptides of more than about 20 amino acid residues. As used herein, the terms "protein" and "polypeptide" are used interchangeably. The term "peptide" typically refers to short polypeptides with more than 10 t0 20 residues.

Polypeptide analysis by mass spectrometry is facilitated by the ability to obtain an accurate mass measurement of a group of peptides derived from a protein by fragmentation that occurs at specific amino acid sequences after using specific cleavage enzymes for proteolysis. The principle behind protein identification assumes that proteins of different amino acid sequence will, after proteolysis with a defined protease, produce a collection of peptides the masses of which constitute protein mass fingerprints unique to a specific protein. If a sequence database containing the specific protein sequence is searched using selected masses based on the experimentally and accurately observed peptide mass fingerprint, a lock mass ion for calibration as external references, combined with the fragmentation rules of the protease, then the protein is expected to be correctly identified within the database. The use of lock mass ions enhances the qualitative and quantitative measurements of mass both for analysis of individual spectra and for the processing of data used in sequencing or other peptide analysis.

Of the MS equipment available, MALDI-MS/MS is commonly used for peptide analysis, although others may be used. Aebersold and Goodlett, 2001; Cramer and Corless, Rapid Comm. in Mass Spectrom. 15: 2058-2066, 2001; see Aebersold and Mann, 2003 for other MS instrument combinations. Chemical modification of the N-terminus of a peptide before MS analysis also has been used to improve MS analysis. The incorporation of a quaternary ammonium group at the N-terminus using the reactive N-hydroxysuccinimidyl ester enhanced sensitivity in MALDI MS (Bartlet-Jones, et al., Rapid Comm. Mass Spectrom. 8: 737, 1994). Cardenas, et al reacted peptides with N-succinimidyl-2-(3-pyridyl)acetate, followed by liquid chromatography separation and analysis by ESI-MS/MS (Cardenas, et al., Rapid Comm. Mass Spectrum. 11:1271-1278, 1997). This reaction modified the N-terminal amino acids and the amino group of lysine. Keough et al. reported the addition of a sulfonic acid group to the N terminus of tryptic peptides increases fragmentation sensitivity and produces much higher fragment ion yields than native peptides. (WO 02/08767; 2003/0032056; WO 02/095419; PNAS 96: 7131-7134, 1999; Rapid Commun. Mass Spectrom 15: 2227-2239, 2001). Destabilization of amide bonds by protonation of amide nitrogen produced extensive fragmentation under MALDI and ESI ionizing conditions (AP MALDI in combination with ion trap MS). The MS/MS spectra of sulfonized peptides containing aspartic, glutamic and oxidized methionine showed more uniform fragmentation along the peptide backbone. Additionally, Keogh, et al. observed the preferential fragmentation on the N-terminal side of proline residues, enhancing recognition of proline.

Chemical modification of the C-terminal amino acid of the peptide before analysis has been found to form longer, more stable series of y-ions. Several methods of C-terminal chemical modification have been reported for lysine. As noted above, trypsin digestion is routinely used in polypeptide analysis by MS to produce fragmentation because the resulting fragment will reliably end in arginine (R) or lysine (K), thus establishing the C-terminal moiety. Although arginine is known to produce an exceptionally strong MS signal, lysine can be difficult to detect. However, lysine can be chemically modified to improve its signal (see Peters, WO 03/056299). This modification distinguishes the mass of lysine from that of glutamine. Cagney and Emili (2002) used a similar approach by differential guanidination of C-terminal lysines followed by LC-ESI-MS/MS analysis (Cagney and Emili, Nat. Biotech. 20: 163-170, 2002). Gu et al (Gu et al., J. Am. Soc. Mass Spectrom. 14: 1-7, 2003) utilized a method incorporating deuterium-labeled (heavy) lysine.

Peters et al. (Peters, et al., WO 03/056299) described a different chemical derivatization method for C-terminal lysine and demonstrated that when the polypeptide's C-terminal lysine was modified by a particular class of reagents, for example 2-methoxy-4,5-dihydro-1-H-imidazole (referred to as "imidazole"), the complexity of the resulting MS/MS spectra was greatly reduced. Peters et al. noted that the y-ion series identification was improved thereby permitting assignment of amino acid sequences more accurately.

Simplification of MS/MS spectra by chemical derivatization of peptides, the additional lock mass ions, and the subsequently improved ability to measure and identify the amino acid sequence data, illustrates the potential for developing high quality fragmentation spectra, obtaining long series of accurate measurements of complete b, and especially y-ion series, and offers a practical approach to *de novo* sequencing. An improved resolution and accurate calibration in *de novo* mass measurements increases the accuracy of sequence determination, and decreases reliance on predictive *in silico* sequence analysis of proteins.

Protein identification by this method involves a few basic steps. Peptides are generated by digestion of the sample protein using amino acid sequence-specific cleavage reagents that allow the residues at the carboxyl- or amino-terminus to be known with a reasonable degree of certainty. For example, the enzyme trypsin leaves arginine (R) or lysine (K) at the carboxyl-terminus of digestion fragments. Accordingly, the N-termini of tryptic peptides (except for the N-terminal one) may be identified as the amino acid following a K or R residue in the protein sequence. Following digestion, the masses of peptides or polypeptides are measured as accurately as possible in a mass spectrometer. The experimental protein fragment mass data are run through a computer and compared with data in a computer database and using the rules that apply to the proteolytic method used in the experiment to generate a list of theoretical masses that are compared to the set of measured masses. An algorithm is used to compare the set of measured peptide masses against those sets of masses predicted for each protein in the database and to assign a score to each match that ranks the quality of the matches. This approach is frequently called *"in silico"* digestion and the correct protein identification by mass analysis depends on the correlation of the measured masses with corresponding data contained in a database. However, several difficulties exist with this approach. Obviously, for a protein to be identified its sequence has to exist in the sequence database being used for comparison. Also, digests of protein mixtures present a problem for mass analysis because it is not readily apparent which peptides in the complex peptide mixture originate from a specific protein. An increase in accuracy of measurement will decrease the potential error for matching an experimental mass to a corresponding mass in a sequence database, and therefore will increase the stringency of the database search.

If a pure protein is digested, and the resulting peptide masses are compared with the list of peptide masses predicted for that protein, two observations are typically made. First, not all of the predicted peptides are detected. Second, some of the measured peptide masses are not present in the list of masses predicted from the protein. The first problem, the missing masses, is usually due to a number of problems that can occur both before and during mass spectrometric analysis such as poor solubility, selective absorption, ion suppression, selective ionization, very short or very long peptide length, missed or inappropriate proteolytic cleavage or other artifacts that cause sample loss or make specific peptides poorly detected or undetectable by MS. This is a critical drawback because missing peptide masses may contain meaningful biological information. Unfortunately, it is not possible to distinguish between trivial and meaningful missing masses without further experimentation. Therefore, unassigned peptide masses are a significant problem for protein identification by mass analysis and probably the single biggest source of misidentifications or missed identifications.

Fragment ion spectra are generated by a process called collision-induced dissociation (CID) in which the amide bonds of a peptide are broken, followed by recording of the fragment ion spectrum. Cleavage of amide bonds results in b-ions (containing the N-terminal) and y-ions (containing the C-terminal). High quality MS/MS spectra of tryptic peptides typically show prominent b and y-ion series. If only these two ions were produced for every amide bond in a 10 residue peptide, the fragment ion spectrum would contain 18 peaks. Ideally, long stable ion series of predominately either the b or y-type would be recovered. In reality, peptide fragmentation is variable and moiety dependent, which leads to gaps and difficulties in analysis. Determining the identity and sequence of a peptide from its MS/MS spectrum is complicated both by the variety and variability of the fragment ions produced. Factors that complicate interpretation of MS/MS spectra are missing ion subsets, internal rearrangements, subsequent fragmentations, and multiple charge states. Also to be considered are the relationship of fragment ion peak intensity to ion series origin and fragment masses, influence of amino acid residues and their derivatives, on neighboring amide bond cleavages, and the link between amino acid composition and neutral loss fragmentation.

Mass spectrometry can define the characteristic of a polypeptide sequence or to determine differences between two forms of a protein or a polypeptide sequence. A comparison of protein expression from two biological conditions, e.g., from cancerous versus normal cells, can lead to the discovery of a protein or set of proteins that are unique to the cancerous state. The ability to use mass spectrometry in proteomics to obtain *de novo* sequence information requires highly accurate MS techniques, reliable generation of MS/MS spectra, and the ability to interpret peptide fragmentation to thereby yield a large number of specific residue identifications leading to sequence information that is truly reliable. To achieve this, several known problems in the use of MS data to determine the sequence of peptides must be overcome. Pursuant to this invention, polypeptides are multiply derivatized to manipulate the fragmentation characteristics such that y-ions in the resulting MS/MS spectra exhibit more nearly equal intensities with minimal gaps and non-sequencing data points compared to other polypeptides that are not so derivatized and are combined with lock mass ions for combined mass analysis.

An important parameter in *de novo* sequencing includes the directionality of fragment ions of polypeptide fragment ion charge retention on the amino (b-ion) or carboxyl (y-ion) terminus. Once directionality of fragment ion orientation is assigned, peptide sequence may then be derived *de novo* by determining the mass for a particular amino acid residue. The *de novo* sequence information produces an extended, reliable identification of individual residues corresponding to a greater part of the entire peptide and enhances the analysis capability when *de novo* data is used in database searching. Sequences derived *de novo* can be compared to those found by database searching and can also be used to analyze the difference per se between experimental and theoretical data. Where the *de novo* sequence differs from the sequence derived by database searching, the difference may be attributable to a biological phenomenon that may be identified in the sample, i.e., a biological sample, containing the polypeptide whose sequence is determined experimentally. The specific peptide-based analyses that may be performed are any of those known techniques where a particular molecular form can be determined based on mass either alone or in comparison with the measurement of a lock mass ion. Molecular forming that may be identified include phosphorylation, acetylation, oxidation, nitration, methylation, silation, glycosylation, crosslinking, etc.

Although specific sequence examples are shown below for MALDI/Q-TOF analyses, those skilled in the art can appreciate that this approach is extensible to other MS interfaces (by way of example, electrospray ionization MS), additional MS ionization schemes, fragmentation approaches, and mass spectrometers. For the example shown, methylation results in the transformation of amino acid side chain carboxyl groups in the C-terminal lysine imidazole-derivatized peptides. The removal of carboxylate group ionic charge could increase the energy required for breaking the adjacent peptide bonds during fragmentation, and thereby, produce MS/MS spectra with improved y-ion intensity distributions. The ability to manipulate the sample to encourage particular fragmentation characteristics and the comparison of experimentally obtained mass data with lock ion mass data greatly simplifies the *de novo* sequence identification (i.e., the "calling" of the linear amino acid sequence).

The present invention improves post-sequencing analyses of peptide data derived from multiply derivatized polypeptides enabled by the disclosure herein. In some cases, the present invention improves the quality of post-sequence data analysis that can be performed. In other cases, the improvement in spectra data quality enables novel techniques that are not currently achievable due to the inherent difficulties in conducting mass analysis of peptide sequences.

Although the present invention discloses specific derivatization strategies, those skilled in the art can envision additional chemical modifications, at the acidic residue side chains, or elsewhere in the polypeptide chain, at various functional groups, in order to generate improvement in *de novo* sequence calling accuracy.

Recently, MS/MS based methods including isotopic labeling and chemical derivatization have improved MS spectral readout (reviewed in Cagney and Emili, 2002). The use of ¹⁶O/¹⁸O labeling improves identification of y-ions, but also reduces the signal intensity (Munchbach et al., Anal. Chem. 72: 4047-4057, 2000; Uttenweiler-Joseph et al., Proteomics 1: 668, 2001). An alternative approach involves methyl esterification of the carboxyl groups in a peptide (Hunt, et al., PNAS 83: 6233, 1986; Goodlett, et al., Rapid Commun. Mass Spectrom. 15: 1214, 2001.) This reaction increases the mass for aspartic and glutamic acid carboxylic side chains, and also modifies the C-terminal carboxyl group. However, for both isotopic labeling and methylation, the modified spectra must still be compared with the original, underivatized peptide spectra. Accordingly, that chemical labeling of peptides may require additional experimental and computational steps that may slow down high-throughput sequencing. Mass spectrometry (MS) involves the analysis of ionized analytes in a gas phase using an ion source that ionizes the analyte, a mass analyzer that measures the mass-to-charge (M/Z) ratio of the ionized analytes, and a detector that registers the number of ions at each m/z value. The MS apparatus may also be coupled to separation apparatus (e.g., such as chromatography columns, on-chip separation systems, and the like) to improve the ability to analyze complex mixtures.

Although the multiple derivatization species and lock mass ion techniques are specially designed to facilitate *de novo* polypeptide sequencing using tandem MS/MS, their application extends to any mass analysis where information derived from mass from a polypeptide is improved by serial derivatization as described below. Also, although certain techniques are described as preferred, for example the derivatization of lysine and the alkylation of carboxyl groups in acidic residues, numerous other derivatizations are contemplated. Of course, the designation of a specific derivatization as either the "first" or "second" in series may be completely arbitrary, and does not exclude simultaneous labeling of two discrete chemical groups on a polypeptide if reaction conditions permit.

In the description of the invention having the use of an isotope tag to yield an isotopic analogue of the species is not considered a derivatization of the present invention, although multiply derivatized polypeptides may also be labeled with isotope tags. In one aspect, multiple derivatization excludes the use of a single labelling species together with a protecting group. Under such circumstances, only a single target moiety on a polypeptide is labelled, but the protecting groups distinguish certain chemical environments allowing a differential quantitation based on the presence of a single label.

On the contrary, in a multiple derivatization strategy, at least two discrete labeling steps are used to independently derivatize two chemical groups of the target polynucleotide, preferably by performing a labeling reaction at substantially all of the available sites for two or more labels. An example of a first derivatization is provided by Peters et al. PCT/US02/35581, WO 03/056299, which is specifically incorporated by reference herein in its entirety. Typically, the sample containing an intact protein, protein or polypeptide fragment, or other polypeptide analyte is cleaved by a chemical reaction that breaks the amide bond of the polypeptides.

Although the description herein uses a trypsin digestion for illustrative purposes, other specific digestions are possible, including but not limited to chymotrypsin, endoproteases, Arg C or Lys C, chemical fragmentation methods, such as the cyanogen bromide cleavage, hydroxylamine cleavage, BNPS-Skatole, etc. However, the trypsin (or endoprotease) cleavages are preferred because the resulting polypeptides feature a C-terminal lysine or aginine residue. USP 5,821,063 provides digestion methods generally for polypeptides. Of course, the derivatization of lysine residues occurs at both terminal and internal lysines, although the labeling of terminal lysines is particularly valuable for sequencing purposes.

In one aspect, lysine residues are derivatized by attaching an imidazole derivative having any of the following formulas: 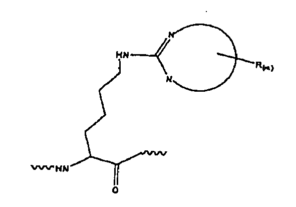
where each R is a functional group independently selected from hydrogen, deuterium, halogen, hydroxyl, cyano, optionally substituted alkyl, optionally substituted alkylcarbamoyl, optionally substituted alkoxy, optionally substituted alkoxycarbonyl, optionally substituted aryl, optionally substituted aryloxy, optionally substituted aryloxycarbonyl, optionally substituted arylcarbamoyl, optionally substituted siloxanly and an affinity tag.

The index "m" is an integer from 0-7, wherein the circle joining the two nitrogens represents an optionally substituted monocyclic or bicyclic ring system having between 2 and 12 additional ring atoms, and wherein the ring atoms are each selected from carbon, oxygen, nitrogen, sulfur and silicon, wherein the foregoing ring atoms are optionally substituted.

In one aspect, the label has the formula 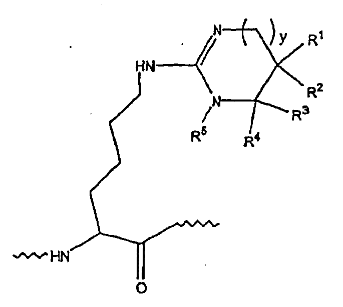
wherein R¹, R², R³ and R⁴ are each functional groups independently selected from hydrogen, deuterium, halogen, hydroxyl, cyano, optionally substituted alkyl, optionally substituted alkylcarbamoyl, optionally substituted alkoxy, optionally substituted alkoxycarbonyl, optionally substituted aryl, optionally substituted aryloxy, optionally substituted aryloxycarbonyl, optionally substituted arylcarbamoyl, and an affinity tag; or in an alternative embodiment, R², R³ and the carbons to which they are attached, join to forma n-membered carbocyclic, heterocyclic, aryl or heteroaryl ring, wherein n is an integer from about 4 to about 8. Preferably, a 5-or 6-membered ring is formed. However, in certain embodiments, y is 0, and its adjacent carbon atom together with R¹ and R² are absent, to form a 4-membered ring.

R⁵ is selected from hydrogen, halogen, hydroxyl, optionally substituted alkyl, optionally substituted alkoxy, optionally substituted aryl and an affinity tag. In Formula I. the index "y" is 0, 1 or 2.

In another aspect, a compound of the following formula is used as a derivatizing agent: 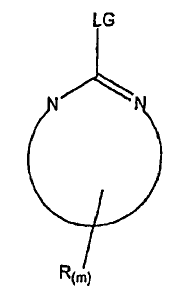
wherein each R is independently a member selected from the group of hydrogen, deuterium, halogen, hydroxyl, cyano, optionally substituted alkyl, optionally substituted alkylcarbamoyl, optionally substituted alkoxy, optionally substituted alkoxycarbonyl, optionally substituted aryl, optionally substituted aryloxy, optionally substituted aryloxycarbonyl, optionally substituted arylcarbamoyl, optionally substituted siloxanly and an affinity tag.

The index "m" is an integer from 0-7, wherein the circle joining the two nitrogens represents an optionally substituted monocyclic or bicyclic ring system having between 2 and 12 additional ring atoms, and wherein the ring atoms are each selected from carbon, oxygen, nitrogen, sulfur and silicon. In Formula II, LG is a leaving group.

In one embodiment, the label has the formula: 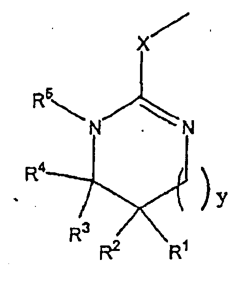
wherein R¹, R², R³ and R⁴ are each independently selected from hydrogen, deuterium, halogen, hydroxyl, cyano, optionally substituted alkyl, optionally substituted alkylcarbamoyl, optionally substituted alkoxy, optionally substituted alkoxycarbonyl, optionally substituted aryl, optionally substituted aryloxy, optionally substituted aryloxycarbonyl, optionally substituted arylcarbamoyl, and an affinity tag; or, in an alternative embodiment R², R³ and the carbons to which they are attached, join to form a n-membered carbocyclic, heterocyclic, aryl or heteroaryl ring, wherein n is an integer from about 4 to about 8. Preferably, a 5-or 6-membered ring is formed. However, in certain embodiments, y is 0, and its adjacent carbon atom together with R¹ and R² are absent, to form a 4-membered ring.

R⁵ is selected from hydrogen, halogen, hydroxyl, optionally substituted alkyl, optionally substituted alkoxy, optionally substituted aryl and an affinity tax. LG is X-CH₃, wherein X is a heteroatom such as O and S. The index "y" is 0, 1 or 2.

A particular embodiment of the above formulas are 2-methoxy - 4.5- dihydro-1H-imidazole and a practice of the above derivatization yields an imidazole derivative at the C-terminal lysine residue of polypeptide digested by trypsin.

The first derivatization is not limited to those that focus on labeling of C-terminal residues to yield improvements in predominantly y-ion spectra. Carderas et al. Rapid Comm. Mass Spectrum. Vol. II, 1271-1278 (1997) labeled peptides prior to passage through an LC column and subsequent analyses by ESI MS/MS analysis. The derivatization reaction is performed in a conventional LC apparatus where protein sample was subjected to modified tyrosinc digestion and then derivatized in-line with N-succiusmidyl-2(3-pyridyl) acetate (SPA). The resulting pyridylacetyl derivative of N-terminal and lysine side-chain amino groups coexisted with partial labeling of trypsin-OH groups. This technique helps distinguish isobaric residues and an alteration of the CID fragmentation pathway in favor of b-ion formation.

An additional functional derivatization of the N-terminal residues of a peptide is described in Bhikhabbai et al. PCT/US02/16247 wherein an aqueous phase derivatization is achieved with an acidic reagent with a sulfonyl moiety together with an activated acid moiety. The features of this reaction are such that it requires a larger sample size due to its tendency to reduce sensitivity of MS detection. In considering the selection of a derivatization reaction, the ability to cause fragmentation reactions from the C-terminal end of a polypeptide fragment to yield y-ions capable of identifying residues in a sequencing analysis must be balanced against the tendency for such derivatizations to dramatically reduce the sensitivity in the resulting mass spectra. The derivatization of Bhikhabbai et al. may be achieved in combination with a step that protects reaction of certain functional groups that would otherwise be derivatized. The combination of a sulfonyl moiety together an activated acid moiety will cause the sulfonation reactions at each lysine residue. In order to protect lysine residues against this reaction, a protection procedure using a guanidation reaction is conducted to specifically protect lysine side chains from reaction in the derivatization step. Such a protecting group reaction is necessary for this species of derivatization in particular where a trypsin digest is used, thereby creating multiple lysine or arginine residues at the C-terminus of the peptide fragment. The combined use of a protecting group and an activated acid moiety together with a sulfonyl moiety is a single derivatization within the context of a multiple derivatization as described herein.

An additional single derivatization is described in Keough et al. (WO 00/43792) wherein a derivatization of the N-terminus of a polypeptide with one or more acidic moieties with a pKa value less than 2 is achieved with, for example, a sulfonic or disulfonic derivative. This derivatization attempts to cause selective cleavage of the amide bonds of the polypeptide in a charge-site specific manner to enable selective detection of only y-ions in a single series.

As noted above, the second derivatization step helps to resolve uniquely problematic mass measurements and detects problems in singly derivatized polypeptides. In one aspect, the second derivatization step comprises alkylation of carboxylate groups in acid side. In one embodiment, the second derivatization step alters the fragmentation characteristics of the derivatized peptide to give a predominantly y- ion series with nearly equivalent intensities. In any of these embodiments, the lock mass ions are introduced prior to the mass measurement and analyzed together with the multiple derivatized polypeptides.

Alkylation of carboxyl groups of acidic amino acid side chains in glutamic and aspartic acid and derivatives and analogues may be achieved as described below in Example 1. The alkylation of the carboxyl groups in a peptide helps distinguish y ions from any other ions present including chemical noise. In the example of a methylation, the reaction also increases the mass of the polypeptide fragment by 14 mass units for each carboxyl group. Absent the acidic side chains of aspartic and glutamic acid, only the C-terminal carboxyl group will be observed to react and exhibit the 14 mass unit shift.

Generally, the alkylation labels the carboxyl groups to form an ester with a straight chain, branched, or tertiary alkyl group of the formula

CH₃(-CH₂)ₙ

when n=0-3 and where the alkyl species may be a methyl, ethyl, propyl, n-propyl, isopropyl, butyl, n-butyl, isobutyl, sec-butyl, or t-butyl species, and where the methyl species is preferred.

The alkylation reaction adds an alkyl group, +14 au for a methylation, to the carboxyl group of the acidic side chains of proteins. This reaction occurs particularly with aspartic acid, glutamic acid, and S-carboxymethylated cysteine. The reaction causes both mass change corresponding to the number of acidic side chains and the species of alkyl group selected and improves the MS/MS spectra. A digestion or other fragmentation may be performed on both derivatized or un-derivatized polypeptides to locate the acidic residues. Accordingly, as noted above, the term "alkylation" or "methylation" typically refers to forming an alkyl or methyl ester of the carboxyl group, however the reaction might not always result in an esterification and the alkylation can also cause alterations in charge distribution around the carboxyl group that still provide the benefits of the present invention without being strictly limited to formation of the alkyl ester.

As will be appreciated by the foregoing description, the invention also includes methods for: derivatization of peptides, mass analysis of multiple derivatized peptides and lock mass ions, determination of amino acid sequence of derivatized peptides, analysis of sequence, and several other specific methods based on the use of data obtained from serially derivatized peptides. An initial step in these methods may include separating and preparing analyte for mass analysis. Typically, this step involves obtaining a sample containing a polypeptide, separating the polypeptide from the sample (although for some samples this step may be omitted), and preparing the polypeptide for the derivatization step by purification, digestion, or otherwise. The analyte is then subjected to at least the first and second chemical derivatizations as described above. The steps may be performed simultaneously if the reactions do not compete or compromise the labeling of the peptide or comprise the structure or chemical composition of the analyte. Once the sample/analyte is prepared, the lock mass ions are introduced as described herein, the mass analysis is performed, and a spectrum is obtained wherein polypeptide fragments and lock mass ions are measured by MS/MS and mass/charge data for the derivatized polypeptides and lock mass ions is obtained. The mass spectrum is comprised of data that correlates the mass/charge ratio of peptide fragments to an amino acid sequence and may be comprised of qualitative or quantitative data in any form or format that may be used together with lock mass ion measurement to assign information about the analyte, which include determining at least a partial an amino acid sequence.

In additional to literal sequence data, the spectra may also contain data reflecting non-sequence information regarding the underlying peptide, including chemical information for the peptide, including gylcosylation, hydration, or other chemical modification. Non-sequence information for a first analyte can be used to determine information about the first analyte directly or can be compared with sequence or non-sequence information from a second analyte or from the nature of the samples from which a first or second analyte is obtained. This type of data analysis is particularly useful when comparing the form of two analyte peptides in proteomics analyses.

The specific techniques include: measuring the experimental or actual mass of an analyte, determining the amino acid sequence of an analyte, introducing lock mass molecules that are ionized within the system such that the lock mass ions are introduced together with the analyte, measuring the mass to charge ratio of the lock mass ions for calibration or reference values, measuring a difference between the experimental or actual mass and a theoretical value based on the molecular weights of the constituent atoms and determining the source of the difference between the experimental values obtain and the theoretical values or known mass data for any polypeptide species.

Mass analysis data or spectra may be used with known sequencing algorithms to yield the amino acid sequence of the peptide analyte (Taylor and Johnson, Rapid Communications in Mass Spectrometry, 11, 1067-1075, 1997; Chen, et al., Journal of Computational Biology, 8(6), 571-583, 2001; Dancik, et al., Journal of Computational Biology, 6, 327-342, 1999; Eng, et al., J. Am. Soc. Spectrom., 5:976-989, 1994; Mann & Wilm, Anal. Chem., 66:4390-4399, 1994). These algorithms are well known and can be used with some degree of utility regardless of the accuracy or precision of the mass analysis data. The improvement in data acquisition and mass spectra quality provided by the present invention increases the accuracy of each mass measurement and increases the utility of sequencing algorithms, increases the accuracy of the sequence information and the length of the polypeptide sequence that can accurately be determined. The methods of the present invention include applying available sequencing algorithms to the sequence information obtained from mass analysis of serially derivatized polypeptides, and securing sequence information for the uniquely derivatized polypeptides or fragments.

Using accurate amino acid sequence data determined using the present invention, the identification of partial and full length proteins can be made from only an accurate determination of a partial amino acid sequence and a search of a protein database. In many proteomics studies and basic biological assays, the critical determination is an identification of the identity of an analyte protein, sometimes as present in a biological sample. Typically, these proteomics databases operate by aligning an experimentally-determined amino acid sequence against a large number of reference amino acid sequences in a database of full-length proteins and identified protein fragments. As is readily appreciated, an increase in the accuracy of sequence information and in the number of sequences identified in a polypeptide analyte will improve the utility of comparing or identifying experimentally-determined polypeptide fragments against reference sequencing. Accordingly, one aspect of the invention is the use of sequence data obtained from mass analysis of the loss mass ions and the derivatized polypeptides described herein to identify proteins by submitting the amino acid sequence, determined from experimental MS data, to a protein database to identify the analyte and/or to identify the analyte as a component of a sample.

It has been shown that five or more amino acid sequences in series (contiguous sequence with no gaps) can be used to search databases to identify a protein with high confidence (Mann & Wilm, Anal. Chem., 66:4390-4399, 1994). These lengths of amino acid sequence have been referred to as critical length sequence tags. Longer amino acid sequence tag could dramatically increase identification accuracy, which is very useful, when many proteins in the database share certain amounts of evolutionarily conserved sequences. Longer amino acid sequence tags also increase the confidence of protein identification for organisms without fully or adequately sequenced genomes. However, when a gap is found in a sequence tag, (for example, instead of a five consecutive amino acid tag, there is a three amino acid tag plus a gap of variable length, followed by a two amino acid tag), the protein identification becomes very difficult. More proteins can be matched to the smaller sequence tags, and because the directionality of the two small tags is also unknown, the protein identification is very unreliable. Mann and Wilm have proposed that the minimum sequence tag for 85% confident protein identification should be at least three to four contiguous residues, but clearly longer sequence tags are beneficial.

As noted above, the technique of the present invention is particularly useful for the MS/MS analysis of post translational modifications in proteins. These modifications are broadly defined as any alteration in the sequence or chemistry of a polypeptide that occurs after the amino acid sequence has been translated from messenger RNA. Post translational modifications can be particularly important in proteomics analyses and the study of proteins in clinical samples related to disease. Many types of post translational modifications, such as glycosylation, and the others described herein, are known to coincide with particular disease states or may indicate physiological conditions that are clinically important in diagnosis of a patient. In some cases, the ability to improve the mass spectra of a polypeptide fragment using lock mass ions and the serial derivatization methods described in the present invention also allows the detection and identification of a specific post translational modification by direct measurement of the mass of a polypeptide analyte and comparison to an internal or external reference value. Under these circumstances, the mass analysis is experimentally performed to measure the mass of a polypeptide fragment and a lock mass ion and the polypeptide fragment mass is compared with the expected mass of the polypeptide fragment either with or without a post translational modification. For example, the addition of a water molecule as a post translational hydration of a polypeptide fragment would increase the mass by 18, i.e., the mass of the added water molecule. When the mass analysis of a polypeptide fragment yields a number that is 18 units different than the native polypeptide, the post translational modification is identified. A similar analyses can be performed for all types of post translational modifications where a difference in a mass measurement from the native polypeptide compared to the modified polypeptide can be made and where the reference mass number is known.

Similarly, there is considerable significance in the identification of the specific residue within a given peptide sequence which has undergone a post-translational modification. For example, in a peptide which possesses more than one site of potential modification. An example of this would be a peptide sequence that has two potential sites of phosphorylation. In order to identify the unique site of modification, a de novo sequence analysis by MS/MS fragmentation may distinguish between the two potential sites of modification, as the MS/MS fragmentation pattern should exhibit a y-ion shifted by the appropriate mass for the additional mass of the phosphoryl group (80 amu), added to which is the mass of the amino acid residue to which the phosphoryl group is attached. Thus, the MS/MS spectral information includes the lock mass ion, the amino acyl mass-depended shift, in addition to the mass of any attendant modifications. It is apparent that mass shifts between adjacent y-ions which do not coincide with known amino acid masses, are diagnostic of the presence of a modification, including the known or yet-to-be-known post-translational modification.

A similar capability exists where any difference in mass analysis can be attributed to a disease or any physiological condition of clinical interest. For example, where a protein mutation is known to be responsible for a particular disease state, and where the mutation is known and results in a difference in mass from the native polypeptide, or that polypeptide representing a normal or non-disease state, a clinical diagnosis may be made from the mass analysis by comparing the mass of a polypeptide analyte in a patient sample from the known mass in the native or non-disease state. For such an application, the methodology of the present invention need only be modified to include a step where the polypeptide analyte is separated from a patient sample prior to the serial derivatization as described above. Further, data processing of the mass data or spectra includes the step of determining the mass of at least one polypeptide fragment comprised of a portion of the patient sample and comparing that result with the known mass for the non-disease state. A comparison of the patient and normal samples indicates whether or not the disease state is present. Because the serial derivatization of the present invention enhances the ability of tandem MS/MS to perform *de novo* peptide sequencing in a high throughput fashion, the invention also increases the utility of the MS/MS technique for clinical diagnosis and large scale screenings for any detection of polypeptide sequences.

As will be apparent to one of ordinary skill in the art, the increased utility of the present invention in polypeptide sequencing also translates into an increased utility in genomics analyses in the use of polynucleotide databases. Any time a polypeptide sequence is known, theoretical polynucleotide sequences can be determined and searches can be made within known databases for similarity with known sequences, i.e., by BLAST or other known techniques. Within the context of the methods of the present invention, the added utility of determining polynucleotide sequences in performing genomics analyses requires only proceeding from the mass analysis of the lock mass ions and the derivatized polypeptide to a determination of the polypeptide sequence, the determination of theoretical polynucleotide sequences by known techniques, and the use of existing polynucleotide databases to correlate the sequence of a polypeptide analyte to the underlying polynucleotide sequence that codes for either the polypeptide fragment or a full length polypeptide containing the fragment.

As with the examples described above for proteomics research, the ability to detect alterations such as mutations or post translational modifications in a protein sample can be coupled to the underlying polynucleotide sequences that code for the protein to perform genomics research based on the sequence of the derivatized polypeptide. As in the proteomics application, data from an experimentally obtained polynucleotide sequence can be analyzed for differences between the experimentally-determined polynucleotide sequence and a reference sequence can be identified and correlated to a disease or other physiological condition. In each such application, the fundamental advantage provided by the invention is the comparison of the spectra or mass data generated by mass analysis of the serially derivatized polypeptide with a reference value, either a reference value for the mass of a known polypeptide, or a reference value for the sequence of a known polypeptide. Accordingly, a comparison of the data generated by the present invention may comprise a calibration or internal or external reference measurement, comparison of experimentally obtained mass data with a database containing reference mass data, or a comparison of experimentally obtained sequence data with a database containing reference sequence data, or a combination of the two.

### Example

Eight proteins, β-casein (bovine milk), myoglobin (horse heart), cytochrome c (bovine heart), β-crystallin (bovine eye lens), calmodulin (bovine brain), human serum albumin, pyruvate kinase (rabbit muscle) and human transferrin dissolved individually in a buffer contains 8M urea, 100 mM NH₄HCO₃, pH 8.5 with final concentration about 2 mg/ml. About 200 µg of each protein were first reduced with tris(2-carboxyethyl)-phosphine hydrochloride at 37 °C for 30 minutes and reacted with iodoacetamide at room temperature for 30 minutes. The resulting protein solutions were then diluted four times with final urea concentration was 2 M, and trypsin was added at 40:1 and incubated at 37 °C overnight. Digestion reaction was quenched by added small amount acetic acid. Cytochrome c and transferrin were reduced and alkylated as described above. Without dilution, Lys-C was added to the protein solution at 100:1 and incubated at 37 °C overnight and quenched with acetic acid.

To modify the carboxy-terminal lysine of peptides with imidazole, tryptic digest of a protein 30 µl (-10 µg) was mixed with 20µl of 1 M imidazole stock (e.g., 2-methoxy-4,5-dihydro-1H-imidazole at a final concentration of 400mM). The reaction mixture was incubated at 60 °C for 3 hours and stopped with 5 µl of glacial acetic acid. The peptides were then purified over a C18 spin column (Pierce), divided into two halves and lyophilized. One half was dissolved in 50:50 v/v methanol:water, and analyzed by MALDI-MS/MS. To derivatize carboxylate groups, the other half was dissolved in 100 µl of 2M methanolic HCI, as an alkylating agent, and incubated at room temperature for 2 hours (Ficarro et al., Nature Biotechnology, 2002, 20:301-305). The reaction was stopped by lyophilization. The lyophilized peptide mixture was redissolved in 50:50 v/v methanol:water, and analyzed by MALDI-MS/MS. Eight different proteins were tested individually using this method. As will be appreciated by those skilled in the art, to increase protein sequence coverage of individual proteins or to analyze more complex protein mixtures, such as protein complexes, or even total cell lysates, the derivatized peptides could also be separated by single or multidimensional separation techniques, for example liquid chromatography, then analyzed by a suitable mass spectral method, for example by MALDI-MS/MS, or on-line electropray ionization MS/MS. Representative MS/MS spectra from cytochrome, pyruvate kinase, and β-crystallin are shown.

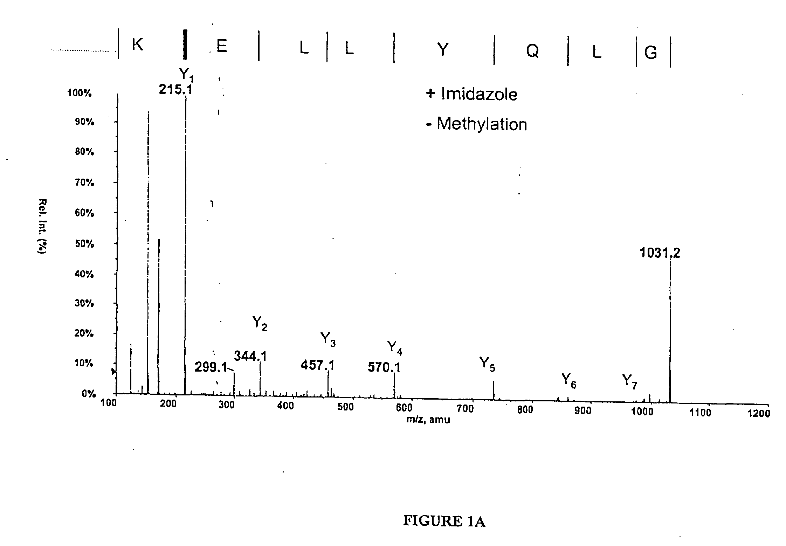

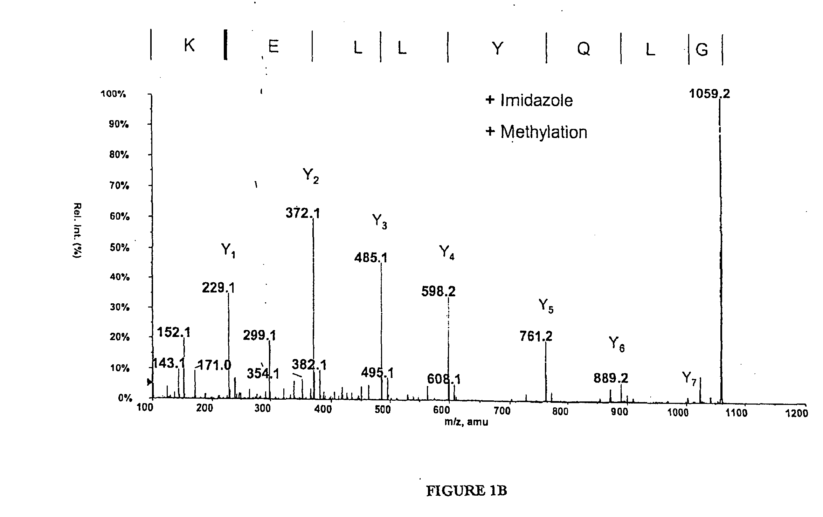

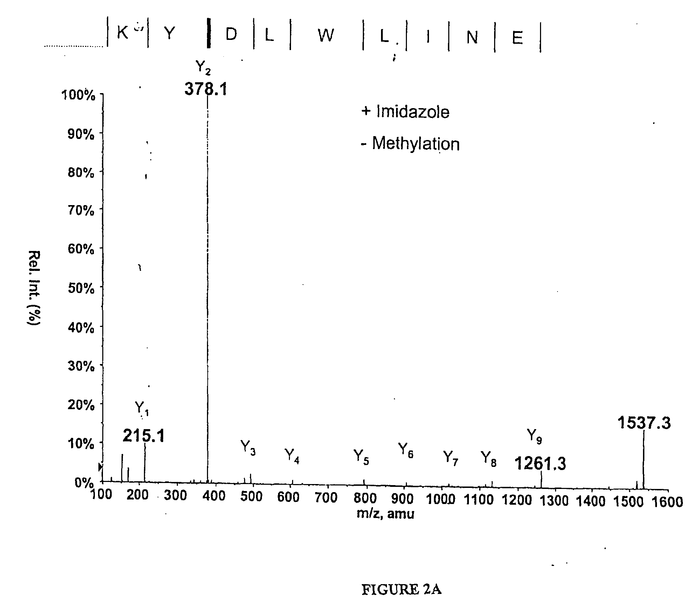

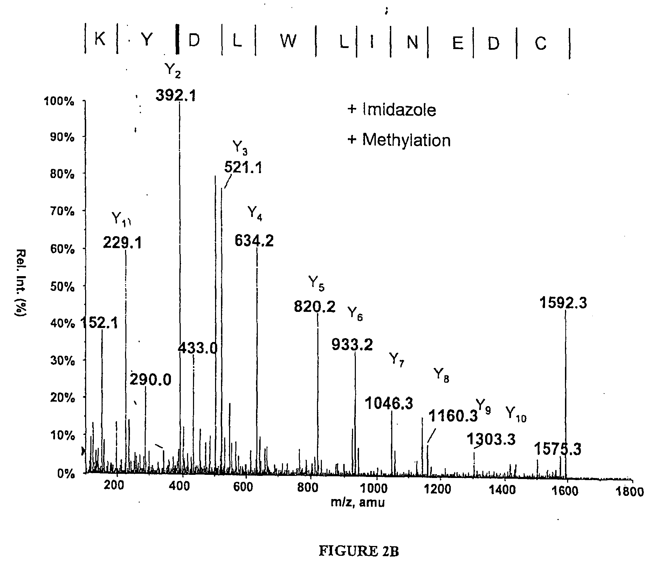

The improvement in spectral quality with the serially derivatized peptides (the B panels of Fig. 1, 2, 3, and 4) is dramatic comparing to the corresponding, non-derivatized peptides (the A panels of Fig. 1, 2, 3, and 4), and peptide sequences can be easily determined from these spectra. In all cases, higher collision energies were required for peptide fragmentation in carboxylate-derivatized peptides than in non-derivatized peptides, an indication of stabilized peptide bonds. The y ions generated by the breakage of carboxylate side chains of acidic residues no longer dominated, such as the y2 ions in panel A of Figure 2. And, in general, the yl ion and its fragments are no longer the dominant features in MS/MS spectra. Both improvements allow more high mass y ions to be detected. Overall, the carboxylate derivatized peptides produced fragments on MS/MS spectra with a more complete y-ion series and evenly distributed peak intensity, a desired feature for *de novo* sequencing.

Referring to Figures 1A and 1B, Fig. 1A is the MS/MS data resulting from a MALDI/Q-TOF MS analysis of a peptide (SEQ ID No: 1) that has been derivatized at the lysine residue using the approach described by Peters, et al (WO 03/056299). As shown in Fig. 1A, certain features might present problems for directly deciphering amino acid sequenced. The y ion and its fragments, i.e. 215.1, 170.1 and 152.1 a.m.u., are dominant in the spectra and have suppressed other y ions, especially those with higher mass. The suppression of other y ions in the series increases the possibility that amino terminal residues in the peptide will be misidentified. In comparison, Fig. 1B shows a substantially improved y-ion intensity distribution and an improved ability to identify the constituent sequences.

An additional problem can result from analysis of polypeptides in which the peptide bond carboxy-terminal to acidic residues, i.e. glutamic acid and aspartic acid, tend to break easily under certain sequence context, resulting in MS/MS spectra with only a few dominant peaks, insufficient for determining the full length sequence of the peptide. This could result in the missed identification of residues of the peptide. This case is exemplified by MS/MS spectral data generated by analysis of a peptide (SEQ ID No: 2) as shown in Fig. 2A. Figure 2B shows the improvement in spectrum quality following a methylation at the polypeptide fragment.

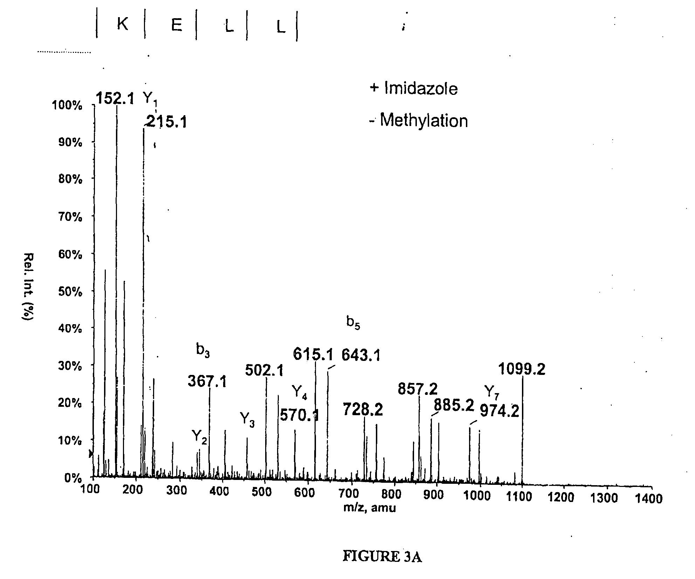

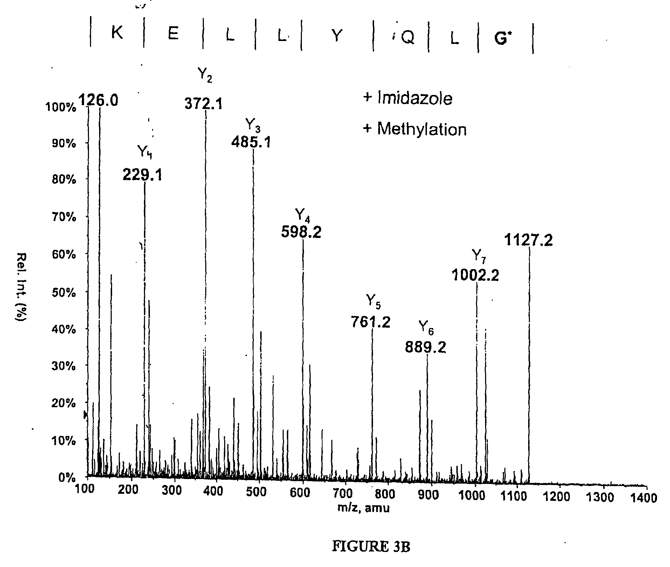

Referring to Figure 3A the MS/MS spectrum resulting from a peptide (SEQ ID No: 1) where both the carboxy-terminal lysine and the amino-terminal glycine were derivatized with imidazole shows that although the primary amines at the amino-terminal of a peptide usually do not react with imidazole reagent, when the amino acid residue at amino-terminal of a peptide is a glycine, the N-terminus is derivatized at a slower rate. The MS/MS spectra from such double-labeled peptide are difficult to interpret *de novo* due to the incomplete y-ions series as well as the presence of y, a, b and some c ions. When the same peptide was serially derivatized pursuant to this intention, the y ion series becomes the dominant feature in the spectrum and *de novo* interpretation became much easier and more accurate, as shown in Figure 3B.

Referring to Figure 4 the MS/MS spectra resulting from a peptide (SEQ ID No: 3) having an internal arginine. Lys C is often used to digest polypeptides to increase the occurrence of C-terminal lysine residues, which increases the ability to use the experimentally-determined sequence for protein identification. However, the internal arginine residue makes the spectrum difficult to interpret, even after imidazole derivatization as shown in Figure 4A. Figure 4B shows the improved MS/MS spectrum from an alkylation at the carboxy groups of the acidic residues of the same peptide, showing the series of amino acid residues leading up to the internal arginine, thereby permitting the determination of a long sequence tag call.

The present invention includes kits containing reagents and instructions for performing the derivatizations described above. The reagents include, but are not limited to, alkylating agents, specifically methylating agents such as methanolic hydrogen chloride, activated imidazole compounds such as 2-methoxy-4,5 dihydro 1H-imidazole, buffers, solvents, and containers for each. The lock mass can be any chemical species that is volatile under reduced pressure and/or elevated temperature levels, chemically stable and ionizable when exposed to photons or ionized reagent gas such as acetone. For example, organic chemicals having molecular weights up to 5000 Da such as fluorinated phosphazines, polyethylene glycols, alkyl amines or fluorinated carboxylic acids may be used. These chemical species are presented by way of example and any number of other equally suitable chemicals may be used in the context of the invention. For example, commonly assigned U.S. Pat. No. 5,872,357 to Flanagan, incorporated herein by reference in its entirety, describes other suitable lock mass materials that can best (be?) used in the manner of the present invention to avoid contamination and charge competition. When organic chemicals are used, it is advantageous to reduce the contribution of carbon isotope C₁₃ to prevent inaccuracies during analysis. Typical organic chemicals used for lock masses have ionization potentials in the range of 7.5 to 12 eV, the majority having ionization potentials below 10 eV, making these chemical particularly suitable for ionization by ultraviolet radiation having photon energies at such levels. The kits may also include reaction vessels, mixing vessels, and indicators to reveal the extent of completion of a chemical reaction. The kits include written instructions to perform the derivatizations described above and to introduce the lock mass ions as described below, and may include instructions for analyzing mass data or mass spectra obtained practicing the present invention. The kits also include solid phase devices for the chromatographic clean-up of reaction products prior to mass spectral analysis.

The data analysis systems include a computer or data processor for analyzing and reporting the mass analysis data, a display unit such as a video monitor and/or a printer to display mass spectra. For sequence analysis, the computer/data processor includes software for performing sequence computations and displaying or printing amino acid sequences. The same or a separate computer/data processor may be used to submit sequence data for database analysis, protein identification, or the proteomic or genomic analyses described above.

The purpose of the internal mass calibration systems described below is to provide a lock mass to the final mass analyzer stage that can be used to correct (calibrate) the mass-to-charge ratio scale of the mass analyzer. In different types of mass analyzers, different scales are used. For example, when a quadrupole analyzer is used, the translation between applied quadrupole voltages and mass-to-charge ratio is calibrated. In a Time-Of-Flight mass analyzer, the translation between ion drift time and mass-to-charge ratio is calibrated. As numerous factors such as temperature, voltage fluctuations, pressure and chamber length affect the calibration in ways that are difficult to calculate and predict, using a reference lock mass is a valuable means of ensuring the accuracy of the mass-to-charge ratios detected and calculated by a mass spectrometer.

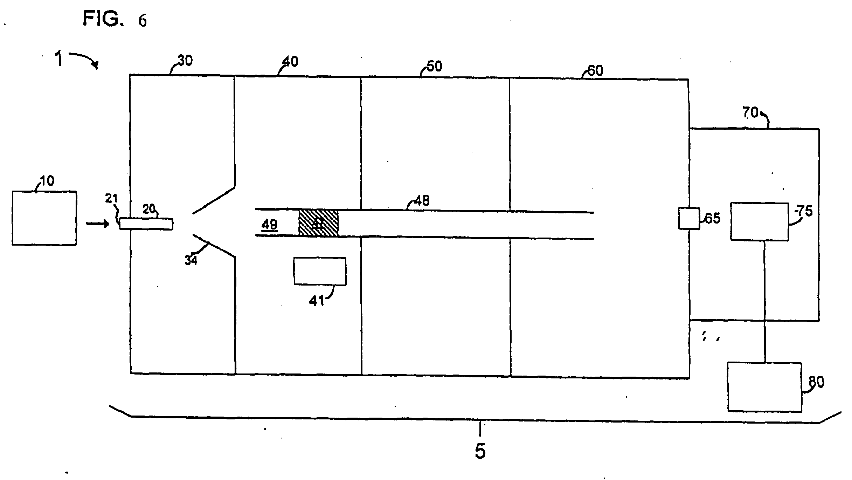

FIG. 6 illustrates an exemplary mass spectrometer system that incorporates the present invention. A mass spectrometer system 1 for analyzing the multiply derivatized polypeptide sample includes an ion source 10 and a mass spectrometer 5. The ion source 10 is used to ionize derivatized polypeptide analyte molecules and to direct the resulting ions toward a mass spectrometer interface 20. Different types of ion sources that may be used in the context of the present invention include Electrospray, Atmospheric Pressure Chemical Ionization, Atmospheric Pressure Photoionization , Matrix Assisted Laser Desorption Ionization, and Atmospheric Pressure-Matrix Assisted Laser Desorption Ionization sources, among other known types. The ion source may be at substantially atmospheric pressure, but sources at pressures lower or higher than atmospheric are considered to be within the scope of use of the invention.

To ensure that a sufficient number of derivatized polypeptide analyte ions enter the mass spectrometer 5 through the interface 20, the source 10 and interface may be maintained at a potential difference that drives the analyte ions toward an aperture 21 in the interface. Other structures or electrodes (not shown) may be present with potential differences that assist in directing the analyte ions in the aperture 21. Gas flow can also be used to assist in driving the ions into the aperture 21. In FIG. 6, the interface 20 is shown as a capillary conduit which extends outward from the mass spectrometer 5 towards the ion source, but it may be just an aperture. The aperture 21 in the interface may typically be in the range 200 - 1000 4m in diameter, but larger or smaller diameters are useable. Additional means not shown may be incorporated into the mass spectrometer 5 or interface 20 to further assist desolvation of the analyte ions. Such means may include a heated capillary which causes solvent to evaporate during transport of the analyte ions within the mass spectrometer, and/or a heated gas counter-flow that dries the analyte ions just before they enter the mass spectrometer via the interface 20. In this manner, a high concentration of ionized analyte relative to the solvent enters the mass spectrometer 5.

Analyte ions pass through the interface 20 and are drawn into a first vacuum stage 30 of the mass spectrometer 5 that is typically at a pressure of approximately 0.5 - 5 torr. Within the first vacuum stage 30, the analyte ions usually undergo a free jet expansion. A skimmer 34 at the downstream end of the first vacuum stage intercepts the jet expansion, and the analyte ions that pass through the skimmer 34 enter into a second vacuum stage 40 that is typically at a pressure of approximately 0.1 to 0.5 torr. It is noted that the vacuum stages 30, 40, 50, 60 depicted in FIG. 6 are coupled to a system of vacuum pumps, as would be understood by those having ordinary skill in the art.

As the analyte ions enter vacuum stage 30, they are driven predominantly by gas flow and voltages on electrodes such as skimmer 34 and other ion optics elements that might be present for aiding transport of the ions. (Such elements that could be present in vacuum stage 30 are not shown in FIG. 6.) Analyte ions that pass through skimmer 34 into vacuum chamber 40 are assisted further in their motion by ion optics 48. In the following, ion optics 48 should be interpreted to include all ion optics elements between interface 20 and mass analyzer 75, including skimmer 34 and other elements in vacuum stage 30 that are not illustrated in Figures 6 - 10.

A source 41 of lock mass ions is located adjacent ion optics 48. "Adjacent" in this context is defined as comprising one or more of the following: "next to", "in the vicinity of", "surrounding", "in part surrounding", "including part of", "connected to", and "functionally associated with". The function of source 41 is to create ions in, or supply ions to, a region 47 that is within ion optics 48. Part of source 41 can thus be located outside of the mass spectrometer vacuum chambers. An example could be a laser or ultraviolet radiation source whose emissions are directed into region 47 through appropriate windows and optics. Another example is a source of lock mass gas that supplies gas into the system and thereby introduces lock mass molecules into region 47 where they can be ionized.

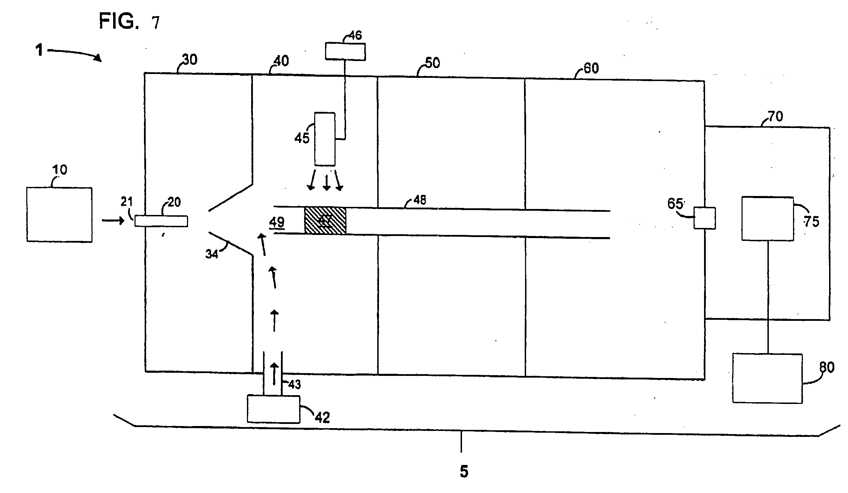

In one embodiment, shown in FIG. 7, lock mass molecules supplied from a lock mass source are introduced in a gaseous phase into the second vacuum stage through an inlet 43. The lock mass can be any chemical species that is volatile under reduced pressure and/or elevated temperature levels, chemically stable and ionizable when exposed to photons or ionized reagent gas such as acetone. For example, organic chemicals having molecular weights up to 5000 Da such as fluorinated phosphazines, polyethylene glycols, alkyl amines or fluorinated carboxylic acids may be used. These chemical species are presented by way of example and any number of other equally suitable chemicals may be used in the context of the invention. For example, commonly assigned United States Patent No. 5,872,357 to Flanagan, incorporated herein by reference in its entirety, describes other suitable lock mass materials that can best used in the manner of the present invention to avoid contamination and charge competition. When organic chemicals are used it is advantageous to reduce the contribution of carbon isotope C₁₃ to prevent inaccuracies during analysis. Typical organic chemicals used for lock masses have ionization potentials in the range of 7.5 to 12 eV, the majority having ionization potentials below 10 eV, making these chemicals particularly suitable for ionization by ultraviolet radiation having photon energies at such levels.

Injected lock mass molecules flow into the second vacuum stage 40 are mixed with derivatized polypeptide analyte ions at a point near to or within the ion optics path 49 of ion optics 48. Within the ion optics path 49, the lock mass molecules become ionized by a lock mass ionization source 45 that irradiates a short span, or ionization region 47, within a single vacuum stage along the axis of the mass spectrometer. The ionization region 47 is confined to a short span along the axis to ensure that lock mass ions have approximately the same collisional conditioning as the analyte ions and are produced at about constant pressure. The radial distance of the ionization source 45 from the central axis depends upon the intensity of radiation it supplies, but in general, the ionization source is placed in close proximity to the ionization region 47 so that maximum radiation is delivered to the region. The ionization source 45 (and ionization region 47) may be situated within the second vacuum stage 40 (as shown) or it may be situated in one of the downstream vacuum stages, e.g., 50, 60. (Collisional conditioning and criteria for location of the ionization source 45 are discussed below.) According to one embodiment, the ionization source 45 is a vacuum ultraviolet (VUV) source, such as, for example, a plasma lamp. Krypton plasma lamps, which produce photons in the range of 10 to 10.6 eV are particularly suitable for the pertinent range of lock mass ionization potentials. Alternatively, a laser ionization technique, such as resonance-enhanced multiphoton ionization (REMPI), may be employed. In either case, a photon flux in the range of 10 9 photons/cm²/s can produce a sufficient ion current required for accurate detection. The ionization source 45 receives electrical power from an external energy source 46. The ionization sources described produce positive lock mass ions by removing electrons from lock mass molecules. Other means of ionization, such as electron impact, can be employed as is known in the art. Alternatively, ionization sources that produce negative lock mass ions by electrical or thermal means may be employed.

According to one embodiment using a photoionization source, a lock mass ionization source 45 is situated within the second vacuum stage 40 in a position that enables photons radiated from the source to intersect with the lock mass molecules within the ion optics path 49. To maximize exposure, it may be advantageous to introduce the lock mass gas at right angles to the central axis of the ion guide 48 and to direct the maximal intensity of the ionization source at right angles with respect to both of these directions. Since photons at energies greater than 7.5 eV tend to become scattered and/or absorbed by background gas components at the pressures prevailing in the second vacuum stage, it can be advantageous to situate the ionization source 45 closely to the ion optics, within a 100 mm range, for example. The ionization source 45 can, however, be situated outside the vacuum system. In that case, the ionizing radiation is transported to the ionization region 47 by means of suitable optics.

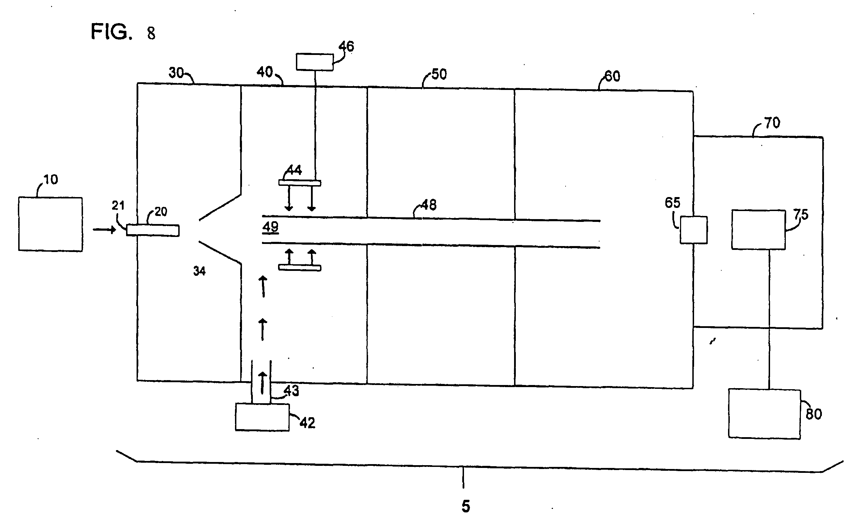

FIG. 8 illustrates an embodiment of the mass spectrometer system according to the present invention in which a concentric VUV lamp is used as the ionization source. In FIG. 8, the concentric VUV lamp 44 is coaxial with, and surrounds a portion of the ion optics 48. As in the previously described embodiment, the axial length of the VUV lamp 44 is limited to a short span in order to define a corresponding ionization region.

Both derivatized polypeptide analyte ions and lock mass ions are guided downstream along the ion optics path 49 defined by the ion optics 48. The optics may include electrodes and circuits that apply electrostatic and/or RF and/or magnetic fields to the ions along the path 49. Typical suitable optics include multipole ion guides such as octopole and hexapole ion guides. Multipole guides can be used in combination with various means known in the art for creating axial electric fields along the ion optics path 49. Suitable guides include, for example, ion funnels such as those described in U.S. Pat. No. 6,107,628.

The ion optics 48 perform at least three functions: first, ion optics move the ions in a generally axial direction and prevent radial loss of the ions between ion source and mass analyzer. Fields generally orthogonal to the axis of the ion optics path 49 serve to confine the ions to regions near the axis, and axial electric fields, often in combination with gas motion, serve to keep ions moving along from ion source to mass analyzer; second, in vacuum staging ion optics assist in stripping off gas accompanying the ions and reduce pressure from about atmospheric in the ion source to about 10⁻⁵ torr or below typical of a mass analyzer. The action of the optics or guides allows the gas to escape into the vacuum chambers and to be pumped away while the ions are constrained to move along the optical path. Typically, a plurality of vacuum chambers is required for the total pressure reduction. The ion optics and/or ion guides facilitate transport of the ions between chambers. The exact number of chambers can vary and is not of importance to the present invention; third, the ion optics or guides cool and focus the ions during transport. In common mass spectrometry practice, collisions of the ions with background gas in an ion guide result in radial and axial cooling and focusing of ions along the axis of the guide. (Focusing in this context means reduction of the radial extent of the beam.) The background gas pressure in the region where this action occurs is typically several millitorr or more. Ion cooling by collision is described in U.S. Pat. No. 4,963,736.

Cooling and focusing are desirable for achieving good resolution and sensitivity with most types of mass analyzers, and especially important for time-of-flight mass analyzers. Substantial ion motion conditioning is necessary for good resolution in TOF analyzers and is achieved by collisional cooling and focusing of the ions before introduction into the analyzer, usually in combination with "slicing" (reduction of the transverse dimensions and divergences) of the ion beam with appropriate apertures. Cooling by reduction of velocity spread of ions, especially in directions transverse to the axis, cannot be achieved with use of ion optics alone (excepting slicing), as a consequence of Liouville's Theorem of constant particle density in phase space.

The motion of an ion particle is described in three coordinates of position x , y , z together with corresponding momentum components pₓ, p_{y}, p_{z}. One such description of motion is the path of the point representing the particle in the 6-dimensional space of the coordinates and the momentum components. This space is called the phase space of the particle. With a system of n such particles, the motion of the system is the set of paths taken by the representative points of the particles in phase space (assuming that the particles do not interact with each other). Liouville's Theorem states: "Under the action of forces that can be derived from a Hamiltonian, the motion of a group of particles is such that the local density of the representative points in the appropriate phase space remains everywhere constant." Forces on ions due to macroscopic electric and magnetic fields external to the ion beam fall into this category. In describing the motion of ions in mass spectrometer systems, coordinate axes can usually be chosen such that the x, y, and z motions are independent of each other. Then each phase space plane (x, p,), (y, p,) and (z, p,) can be considered separately. For this usual circumstance, Liouville's theorem means that regions of each of these planes occupied by representative points of the ions may change in shape, but not in area, as the motions of the ions proceed. The magnitude of the areas can only change by the action of nonconservative forces (e.g., collisions) or by removal of ions from the beam (e.g., slicing).

In the following, "phase space of ions" should be interpreted to mean "the region of the phase space plane that is occupied by the representative points of the ions". The particular phase space plane referred to in the description of the invention is a phase space plane associated with a coordinate axis orthogonal to the longitudinal axis of the ion guide or ion optics. Such orthogonal axes may also be called "transverse".

If the lock mass ions are not cooled and focused in the identical fashion as the analyte ions (i.e., their respective phase spaces transverse to the axis are not essentially congruent), the instrumental mass resolution will likely be different for the two species. Under some circumstances, erroneous mass calibrations could result. It is thus important that the lock mass ions be subjected to substantially the same cooling and focusing as the derivatized polypeptide analyte ions. This is accomplished by creating the lock mass ions in the ion guide before significant cooling and focusing takes place, i.e., before the ions reach a region of pressure appropriate for cooling, nominally about 5 millitorr or greater. The optimal position for ionization of the lock mass molecules in a particular embodiment of the ion optics 48 is thus readily determined by one of ordinary skill in the art.

Thus, to condition the motions of the lock mass ions and the derivatized polypeptide analyte ions in a comparable manner in the example system of FIG. 6, the lock mass and analyte ions are directed along the same ion optics path 49. They are therefore subjected to approximately the same average history of collisions with the background gas. In this example, much of the collisional cooling occurs before the third vacuum stage 50, which is maintained at about 5 millitorr or somewhat less. To facilitate cooling, the third vacuum stage 50 may be longer than the other stages in order to lengthen the ion optic path 49 and thereby increase the probability of collision between the ions and the gas molecules.

From the third vacuum stage 50, the derivatized polypeptide analyte and lock mass ions enter a fourth high vacuum stage 60 in which the pressure drops to less than about 10⁻⁴ torr, or less than about 10⁻⁵ torr in some applications. An interface 65 to a vacuum chamber 70 containing a mass analyzer 75 is positioned at the downstream end of the fourth vacuum stage. Any type of mass analyzer can be used; examples include ion trap, quadrupole mass filter, magnetic sector, TOF, and Fourier Transform Ion Cyclotron Resonance (FTICR) analyzers. Actual choices of pressure near or in the mass analyzer will depend upon the type of mass analyzer used, and will range from greater than 10⁻⁴ torr in the case of an ion trap analyzer to less than 10⁻⁵ torr for an FTICR analyzer, with intermediate values in the cases of quad mass filters and TOF analyzers. If a TOF analyzer is used, the interface 65 may comprise a slicer that is used to limit the transverse extent of the ion beam before entrance to an orthogonal acceleration chamber. Derivatized polypeptide analyte and lock mass ions are selected and then detected with a an ion detector in the mass analyzer 75.

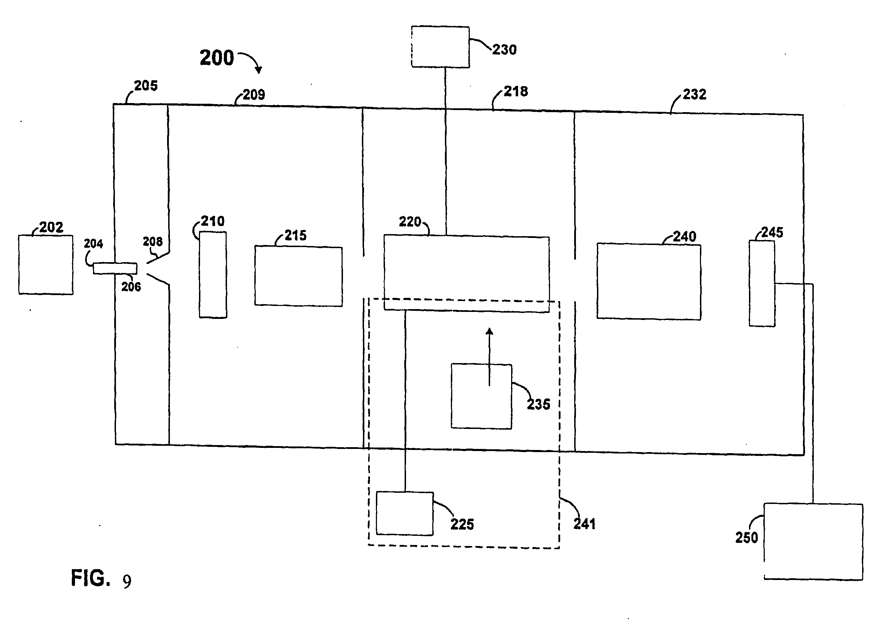

FIG. 9 schematically illustrates an embodiment of a tandem mass spectrometer system 200 that provides lock mass calibration and measurement of derivatized polypeptide analyte. As shown, a source of derivatized polypeptide analyte ions 202 introduces analyte ions into a vacuum interface chamber 205 through an aperture 204 of a longitudinally positioned capillary conduit 206. Analyte ions flow through the interface chamber 205 and skimmer 208 into a first mass analyzer 215 in vacuum chamber 209. Optionally, ion optics 210 are included for focusing and accelerating analyte ions into the mass analyzer 215. Analyte ions within a desired mass range are selected for passage through the mass analyzer, the remainder of the ions being filtered away. The selected analyte ions that travel through the first mass analyzer 215 then enter a collision cell 220 in vacuum chamber 218 after being accelerated to a kinetic energy appropriate for collisional dissociation. In the collision cell 220, at least a portion of the "parent" analyte ions, usually a long polypeptide or protein are fragmented into "daughter" ions by collisions with a gas, which may be an inert gas such as nitrogen, supplied from a collision gas source 230 and maintained at an appropriate pressure. As is known in the art, the collision gas pressure and length of the collision cell 220 are chosen to yield sufficient dissociative collisions to produce a desired amount of daughter ions. The daughter ions, usually shorter length polypeptide fragments, are then transported by gas flow or by ion optics (not shown) to a second mass analyzer 240 in vacuum chamber 232. In some embodiments, the daughter ion transport may be assisted by DC electric fields in the collision cell 220. Lock mass ions are created in, or introduced into, the collision cell 220 from a source 241 of lock mass ions adjacent (in the same sense as described above) the collision cell 220. In some embodiments, the source 241 of lock mass ions may comprise a lock mass source 225 for supplying lock mass molecules to collision cell 220 and a lock mass ionization source 235 for ionizing lock mass molecules within the collision cell 220. The lock mass source 225 may, for example, be a gas source. The lock mass ionization source 235 may be an ultraviolet radiation source or laser, for example. The lock mass ions are transported together with the analyte daughter ions to the second mass analyzer 240, again by means of gas flow, DC electric fields in the collision cell 220, ion optics (not shown), or combinations thereof. The ions enter second mass analyzer 240, which selects lock mass ions and the analyte daughter ions for passage to a detector 245. Data analysis may follow in a data acquisition and processing unit 250 connected to or included within the detector 245.

Analyzers 215 and 240 can be any types of mass analyzer or mass filter. An exemplary embodiment incorporates a quadrupole mass filter at 215 and a time-of-flight mass analyzer at 240. In some embodiments, the first analyzer 215 and collision cell 220 may be combined into a single device that has the functions of both: mass selection and ion fragmentation. Examples include quadrupole ion traps and linear ion traps. An exemplary embodiment of this type could include an ion trap at 215 and a time-of-flight mass analyzer at 240, with optional beam conditioning ion optics in between. A distinct collision cell would then not be necessary. The actual number of distinct vacuum chambers will vary with embodiment.

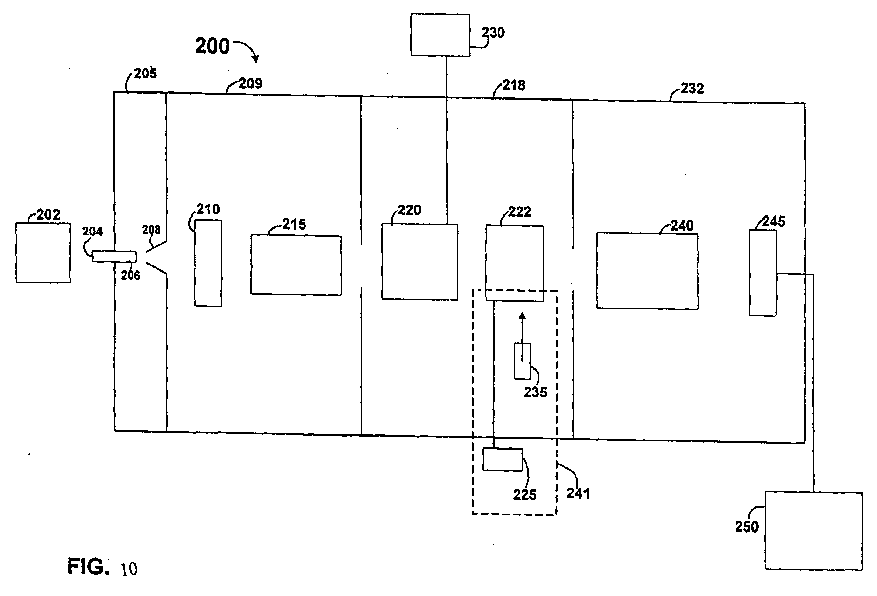

Usually, the lock mass molecules can be introduced anywhere in the collision cell 220 and can be ionized at any or all positions along the longitudinal axis of the cell. Since the lock mass ions will have essentially thermal initial kinetic energy, they will not be subjected to collisional dissociation. For embodiments where fields (DC, AC or RF) within the collision cell 220 are used for dissociation of the analyte ions, it may be advantageous to ionize the lock mass molecules at or near the downstream end of the cell, so that no significant fraction of the lock mass ions is dissociated before leaving the cell. In embodiments where beam conditioning ion optics are placed downstream from the collision cell 220, between the cell and the second mass analyzer 240, lock mass ions can be created in the optics rather than in the collision cell. One such embodiment is illustrated schematically in FIG. 10. Ion optics 222 for beam conditioning are placed between the collision cell 220 and second mass analyzer 240. Lock mass ions are created in, or introduced into, ion optics 222 from a source 241 of lock mass ions adjacent (in the above sense) the ion optics 222. In some embodiments, the source 241 of lock mass ions may comprise a lock mass source 225 for supplying lock mass molecules to ion optics 222 and a lock mass ionization source 235 for ionizing lock mass molecules within the ion optics 222. The lock mass source 225 may, for example, be a gas source. The lock mass ionization source 235 may be an ultraviolet radiation source or laser, for example. The lock mass ions are transported together with the analyte daughter ions to the second mass analyzer 240 by means of gas flow, DC electric fields, the ion optics 222, or combinations thereof. Mass analysis of the ions follows as described above. In some embodiments, first mass analyzer 215 and collision cell 220 may be combined into a single device such as an ion trap, as described above. The scope of the term "collision cell" in the claims includes the embodiments where functions of a collision cell, e.g., ion fragmentation, are performed in another device or apparatus.

Distinct methods of calibrating mass spectrometer systems by internal introduction of lock masses have been mentioned in connection with the several embodiments of mass spectrometer systems described above. According to a first method, lock mass molecules are introduced into a post-source vacuum stage of a mass spectrometer system and then ionized in or near the downstream path of the analyte ions so that both analyte ions and lock mass ions thereafter travel along the same path downstream and are detected and analyzed together. In a second method, for calibrating a tandem mass spectrometer, lock mass molecules are introduced and ionized in the path of analyte daughter ions. The lock mass ions are then guided and transported together with the analyte daughter ions for detection and analysis.

The use of internal lock mass introduction in the exemplary methods described above can provide advantages over introduction into the ion source. Though possible, switching between analyte sample and lock mass solutions is not necessary, and no washout time is required between introduction of analyte and lock mass samples since the lock mass material does not contaminate the ion source or its interface with the mass spectrometer. The throughput and speed of sample analysis is correspondingly increased. All types of ion sources can be employed, without restriction imposed by lock mass ionization requirements or contamination problems. The lock mass molecules and do not react with the derivatized polypeptide analyte and do not compete for ionization.

All publication and patent application cited in this specification are herein incorporated by reference to the extent not inconsistent with the present disclosure as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims.

## Claims

1. A method to perform a mass analysis of a polypeptide comprising:
performing a first derivatization of a polypeptide analyte;
performing a second derivatization of a polypeptide analyte;
ionizing lock mass molecules and the derivatized polypeptide analyte,
obtaining a mass analysis of the lock mass molecules and the derivatized polypeptide.

2. The method of Claim 1 further comprising the step of digesting the polypeptide prior to the first derivatization.

3. The method of Claim 1 or 2 wherein the first derivatization comprises reacting the polypeptide with an imidazole.

4. The method of any preceding claim, wherein the second derivatization comprises reacting to polypeptide analyte with an alkylating agent.

5. The method of Claim 4 wherein the second derivatization yields an alleyl derivative of a carboxyl group of an acidic side chain of glutamic acid or aspartic acid.

6. The method of any preceding claim further comprising determining an amino acid sequence of the serially derivatized polypeptide from the mass analysis.

7. The method of Claim 3 wherein the step of reacting the polypeptide with the imidazole is performed with 2-methoxy-4,5 dihydro 1H-imidazole.

8. The method of Claim 4 wherein the second derivatization comprises methylating the carboxyl group.

9. The method of Claim 4 wherein the step of reacting the polypeptide with the alkylating agent yields an alkyl derivative of the carboxyl group which alkyl is ethyl, propyl, n-propyl, isopropyl, butyl, n-butyl, isobutyl, sec-butyl, t-butyl or any combination thereof.

10. The method of any preceding claim wherein the step of ionizing the lock mass molecule and the polypeptide analyte occurs within the collision cell.

11. The method of any preceding claim, wherein the step ofionizing the lock mass molecules and the serially derivatized polypeptide uses ultraviolet radiation.

12. A method to determine a sequence of a polypeptide analyte comprising:
performing a first derivatization of a polypeptide analyte to yield a derivatized polypeptide analyte having derivatized lysine;
reacting the derivatized polypeptide analyte with a second agent to yield a multiply derivatized polypeptide analyte;
introducing lock mass molecules;
ionizing the lock mass molecules and the multiply derivatized polypeptide; and
performing a mass analysis of the multiply derivatized polypeptide analyte.

13. The method of Claim 12 further comprising the step of determining an amino acid sequence of the multiply derivatized polypeptide analyte from the mass analysis.

14. The method of Claim 13 further comprising comparing the amino acid sequence with a reference sequence.

15. The method of Claim 14 further comprising determining the difference in a mass of the amino acid sequence and a mass of the reference sequence.

16. The method of Claim 15 further comprising correlating the comparison to a post translational modification.

17. The method of any of claims 12 to 16 further comprising the step of digesting the polypeptide analyte prior to the first derivatization.

18. The method of any of claims 12 to 17 wherein the step of ionizing of the lock mass molecules and the multiply derivatized polypeptide occurs within the collision cell.

19. The method of any of claims 12 to 18 wherein the step of ionizing the lock mass molecules and the multiply derivatized polypeptide uses ultraviolet radiation.

20. A method to perform a mass analysis of a polypeptide comprising:
ionizing lock mass molecules and a derivatized polypeptide comprising an imidazole-derivatized lysine group and an alkylated carboxyl group; and
obtaining a mass analysis of the lock mass molecules and the derivatized polypeptide.
